# EUROPÄISCHE PATENTSCHRIFT

(11) **EP 1 525 475 B1**
(45) Veröffentlichungstag und Bekanntmachung des Hinweises auf die Patenterteilung: **22.11.2006**
(21) Anmeldenummer: 03787705.7
(22) Anmeldetag: 21.07.2003
(51) Int. Cl.: G01N 33/543

(54) **SENSOROBERFLÄCHE MIT VERBESSERTEM SIGNAL/RAUSCH-VERHÄLTNIS**
SENSOR SURFACE COMPRISING AN IMPROVED SIGNAL-NOISE RATIO
SURFACE DE CAPTEUR POSSEDANT UN RAPPORT SIGNAL/BRUIT AMELIORE

(30) Priorität: 22.07.2002 DE 10233303
(43) Veröffentlichungstag der Anmeldung: 27.04.2005
(73) Patentinhaber: Micronas Holding GmbH, 79108 Freiburg (DE); Klapproth, Holger, Dr., 79108 Freiburg (DE)
(72) Erfinder: KLAPPROTH, Holger, 79108 Freiburg (DE)
(74) Vertreter: Stürken, Joachim
(86) Internationale Anmeldenummer: PCT/DE2003/002452
(87) Internationale Veröffentlichungsnummer: WO 2004/017070

(56) Entgegenhaltungen:
- EP-A- 0 874 242
- EP-A- 1 215 287
- WO-A-01/94946
- WO-A-98/12539
- WO-A-99/43688
- US-A1- 2002 055 093

## Beschreibung

Die Erfindung betrifft eine Sensoroberfläche mit verbessertem Signal/Rausch-Verhältnis auf der Basis von auf der Sensoroberfläche kovalent immobilisiertem Blockierungsreagenz sowie derartige Oberflächen umfassende Vorrichtungen und Verfahren unter Verwendung derselben.

Bei Sensoren, die nach dem Rezeptor/Liganden-Prinzip arbeiten, z.B. Antikörper/Antigen-Sensoren (Proteinsensoren), wird zumeist keiner der Reaktionspartner oder aber nur der Rezeptor kovalent immobilisiert. Nach dem Immobilisieren (Aufbringen) des Rezeptors wird meist vor der eigentlichen Reaktion ein Blockierungsreagenz oder -agens zugegeben, das verhindern soll, dass Analytmoleküle unspezifisch, d.h. nicht ausschließlich an den Rezeptor, an der Oberfläche des Sensors gebunden werden. Dieses Blockierungsreagenz belegt nicht genutzte Bereiche auf dem Sensor und ist für fast alle Messungen zwingend notwendig, um ein ausreichend hohes Signal/Rausch-Verhältnis zu bekommen.

Ein bei Sensoren des Standes der Technik bisher stets auftretendes Problem ist, dass bei späteren Reaktions- und Waschschritten das Blockierungsreagenz entfernt wird. Eine Entfernung des Reagenzes führt zu einer (partiellen) Freilegung von Oberflächenbereichen auf dem Sensor, an denen dann wieder unspezifische Wechselwirkungen auftreten können. Eine solche Wechselwirkung oder Reaktion würde ein unspezifisches Signal bei der Detektion erzeugen, wodurch das Verhältnis von spezifischem Signal zu unspezifischem Signal deutlich verschlechtert würde.

Die EP-A-0 874 242 offenbart eine Festphasen-Vorrichtung zur Durchführung von Assays, welche ein Substrat und eine Vielzahl von diskreten Reaktionsstellen umfasst, wobei die Reaktionsstellen jeweils einen kovalent an das Substrat gebundenen Liganden aufweisen und die Oberfläche des Substrates zwischen den Reaktionsstellen hinsichtlich des Analyten inert ist. Dies wird erreicht, indem die gesamte Oberfläche aktiviert und geeignete Liganden innerhalb diskreter Bereiche dieser aktivierten Oberfläche kovalent immobilisiert werden, während die anderen aktivierten Oberflächenbereiche mit üblichen, kovalent gebundenen Reagenzien ohne photoreaktive Gruppe blockiert werden können. Obwohl diese Vorrichtung dauerhaft blockierte Oberflächenbereiche aufweist, sind die diskreten Bereiche von diesen nicht umfasst.

Aufgabe der Erfindung ist, diesen den Sensoren des Standes der Technik anhaftenden Nachteil zu beseitigen.

Erfindungsgemäß wird diese Aufgabe gelöst durch die Bereitstellung einer Sensoroberfläche mit darauf kovalent immobilisierten spezifischen Sondenmolekülen für mindestens ein nachzuweisendes Biomolekül, wobei grundsätzlich für unspezifische Bindungen zur Verfügung stehende Stellen oder Bereiche der Sensoroberfläche durch mindestens ein kovalent daran immobilisiertes Blockierungsreagenz inaktiviert sind, erhältlich durch ein Verfahren, bei dem die Sensoroberfläche nach der kovalenten Immobilisierung der Sondenmoleküle mit dem Blockierungsreagenz beaufschlagt wird, wobei das Blockierungsreagenz mindestens einen photoreaktiven Vernetzer mit mindestens einer photoreaktiven Gruppe aufweist und die kovalente Immobilisierung desselben an der Sensoroberfläche durch geeignete Bestrahlung erfolgt.

Kurz zusammengefasst werden also erfindungsgemäß Affinitätssensoren für Biomoleküle, die nach dem Rezeptor/Liganden-Prinzip arbeiten, nach dem Aufbringen der Rezeptormoleküle mit einem Reagenz behandelt, durch das unspezifische Interaktionen von Analytmolekülen außerhalb der für die spezifische Bindung an den bzw. die Rezeptor(en) vorgesehenen Bereiche der Sensoroberfläche durch kovalentes Binden eines sog. Blockierungsreagenzes oder -agens unterbunden werden. Dadurch wird das Signal/Rausch-Verhältnis der Reaktion verbessert und die Empfindlichkeit der Analyse erhöht. Kovalentes Immobilisieren bzw. Binden der Rezeptoren und der Blockierungsreagenzien auf der Sensoroberfläche erlaubt es, die Spezifität der Reaktion durch die Verwendung von z.B. hohen Tensidkonzentrationen zu erhöhen, weil während diverser Waschschritte, die stattfinden, um nicht gebundenen Analyt von dem Sensor zu entfernen, das Auswaschen der Blockierungsreagenzien verhindert wird.

Da das Signal/Rausch-Verhältnis bezüglich der Rezeptoren verbessert wird (d.h. nur das biologische Signal/Rausch-Verhältnis nicht das elektronische Signal/Rausch-Verhältnis), wird auf diese Weise die absolute Sensitivität der Messungen erhöht und dadurch sowohl die Sensitivität des Sensors verbessert als auch die dynamische Breite der Messung deutlich erhöht. Insbesondere ergibt sich durch die Verwendung von lipophilen Photovernetzern bzw. »Photocrosslinkern« und amphiphilen Blockierungssubstanzen die Möglichkeit, Oberflächeneigenschaften gezielt zu verändern, und zwar ohne das Risiko, die wertvollen Rezeptorproteine zu beschädigen oder in sonstiger Weise zu beeinträchtigen. Auf diese Weise können z.B. Antikörperchips gegebenenfalls wiederverwendet werden, da der Aufbau und die Belegung des Chips im wesentlichen aufrecht erhalten bleiben.

Ein weiterer Aspekt der Erfindung betrifft ein Verfahren zur Verbesserung des Signal/Rausch-Verhältnisses von Biosensoren, bei dem eine erfindungsgemäße Sensoroberfläche verwendet wird. Daher betrifft die vorliegende Erfindung grundsätzlich alle Verfahren zum Nachweis des Vorhandenseins von Analyten in einer zu untersuchenden Probe unter Verwendung Oberflächen-gebundener Rezeptormoleküle, bei denen eine erfindungsgemäße Sensoroberfläche verwendet wird.

Ein weiterer Aspekt der Erfindung betrifft eine Vorrichtung zur Verwendung in einem erfindungsgemäßen Verfahren, die eine erfindungsgemäße Sensoroberfläche aufweist.

Ein weiterer Aspekt der Erfindung betrifft einen Kit zur Verwendung in einem erfindungsgemäßen Verfahren, der eine erfindungsgemäße Sensoroberfläche und gegebenenfalls Puffer und Nachweisreagenzien enthält.

Ein weiterer Aspekt der Erfindung betrifft ein Reagenz zur Blockierung zur Verfügung stehender Bindungsstellen mit mindestens einem photoreaktiven Vernetzer mit mindestens einer photoreaktiven Gruppe zur kovalenten Immobilisierung an einer Sensoroberfläche.

Ein weiterer Aspekt der Erfindung betrifft ein Verfahren zur Herstellung eines erfindungsgemäßen Blockierungsreagenzes, bei dem mindestens ein Blockierungsreagenz, wie oben definiert, mit mindestens einem Vernetzer umgesetzt wird, der mindestens eine photoreaktive Gruppe aufweist.

Ein weiterer Aspekt der Erfindung betrifft einen Kit zur Herstellung einer erfindungsgemäßen Sensoroberfläche, der mindestens ein erfindungsgemäßes Blockierungsreagenz der zuvor definierten Art und gegebenenfalls eine Sensoroberfläche sowie Puffer und Reagenzien enthält.

Weitere vorteilhafte und/oder bevorzugte Ausführungsformen der Erfindung sind Gegenstand der jeweiligen Unteransprüche.

Bei einer Ausführungsform der erfindungsgemäßen Sensoroberfläche bilden die Sonden- bzw. Rezeptormoleküle ein adressierbares Muster auf der Oberfläche. Derartige Muster sind aus der Bioarray- oder Biochip-Technik an sich bekannt und können mit beliebigen der dort angewendeten Techniken erzeugt werden, beispielsweise durch Aufdrucken. Die Array-Technik gestattet die parallele Untersuchung einer sehr großen Anzahl von Analyten.

Zur Immobilisierung von Sondenmolekülen können beliebige Techniken angewendet werden, beispielsweise die von G. T. Hermanson in "Bioconjugate Techniques", Academic Press, 1996, beschriebenen. Beispielsweise eignen sich im Falle von aminoterminierten Oligonukleotiden sogenannte reaktive Ester oder Reaktivester wie N-Hydroxysuccinimide (NHS-Ester), Epoxide, vorzugsweise Glycidyl-Derivate, Isothiocyanate, Isocyanate, Azide, Carbonsäuregruppen oder Maleinimide. Selbstverständlich könnte auch mit den gleichen Photovernetzern immobilisiert werden, die weiter unten zur Immobilisierung der Blockierungsreagenzien beschrieben werden.

Bei einer weiteren Ausführungsform der erfindungsgemäßen Sensoroberfläche erfolgt die kovalente Immobilisierung des mindestens einen Blockierungsreagenzes über mindestens einen photoreaktiven Vernetzer oder »Crosslinker«. Es ist möglich, mehrere unterschiedliche Blockierungsreagenzien parallel zu verwenden. Grundsätzlich ist jedes im Stand der Technik verwendete Blockierungsreagenz erfindungsgemäß geeignet. Jedes Blockierungsreagenz kann gegebenenfalls mit mehreren, auch unterschiedlichen, photoreaktiven Vernetzern immobilisiert werden.

Bei einer weiteren Ausführungsform der erfindungsgemäßen Sensoroberfläche weist der mindestens eine photoreaktive Vernetzer mindestens eine unter Benzophenon oder Derivaten davon, Anthrachinon oder Derivaten davon, Thymidin oder Derivaten davon und 4-Azidobenzoesäure oder Derivaten davon ausgewählte photoreaktive Gruppe auf. Grundsätzlich ist jeder photoreaktive Vernetzer des Standes der Technik erfindungsgemäß geeignet.

Bei einer weiteren Ausführungsform der erfindungsgemäßen Sensoroberfläche ist die Sensoroberfläche unter Metall-, Halbmetall-, Halbmetalloxid-, Glas- oder Polymeroberflächen ausgewählt.

Bei einer weiteren Ausführungsform der erfindungsgemäßen Sensoroberfläche ist die Metalloberfläche unter Gold- und Aluminiumoberflächen ausgewählt.

Bei einer weiteren bevorzugten Ausführungsform der erfindungsgemäßen Sensoroberfläche ist die Halbmetalloberfläche eine Siliciumoberfläche.

Bei einer weiteren Ausführungsform der erfindungsgemäßen Sensoroberfläche ist die Halbmetalloxidoberfläche eine Siliciumoxid oder Aluminiumoxidoberfläche.

Bei einer weiteren Ausführungsform der erfindungsgemäßen Sensoroberfläche ist die Glasoberfläche eine Quarzglasoberfläche. Grundsätzlich ist jede bekannte Glasoberfläche erfindungsgemäß geeignet.

Grundsätzlich sind alle Oberflächenformen erfindungsgemäß geeignet. Obgleich die Oberfläche bei Biochip-Anwendungen zumeist im wesentlichen eben ist, liegt es für den Fachmann auf der Hand, dass auch Oberflächen mit Vertiefungen sowie solche, die nicht flach sondern rund bzw. sphärisch ausgestaltet sind, erfindungsgemäß gleichermaßen geeignet sind.

Bei einer weiteren Ausführungsform der erfindungsgemäßen Sensoroberfläche ist die Polymeroberfläche unter Oberflächen aus Cycloolefincopolymer oder Derivaten davon, Polystyrol oder Derivaten davon, Polyethylen oder Derivaten davon, Polypropylen oder Derivaten davon, Polyimid oder Derivaten davon und Polymethylmethacrylat oder Derivaten ausgewählt. Grundsätzlich ist jede bekannte Polymeroberfläche erfindungsgemäß geeignet. Hiervon umfasst sind auch Oberflächen, die quellbare oder wasserpermeable polymere oder copolymere Strukturen aufweisen und mono-, bi- oder polyfunktionalisierte Kopplungsgruppen umfassen können.

Ferner sind die im Stand der Technik für analytische oder diagnostische Zwecke bekannten Membranen wie insbesondere solche aus Nylon und Nitrocellulose geeignet.

Bei einer weiteren Ausführungsform der erfindungsgemäßen Sensoroberfläche ist das Sondenmolekül (Rezeptor) ein Partner eines spezifisch wechselwirkenden Systems von komplementären Bindungspartnern (Rezeptor/Ligand).

Bei einer weiteren Ausführungsform der erfindungsgemäßen Sensoroberfläche beruht das spezifisch wechselwirkende System von komplementären Bindungspartnern auf der Wechselwirkung einer Nukleinsäure mit einer komplementären Nukleinsäure, der Wechselwirkung einer Peptidnukleinsäure mit einer Nukleinsäure, der Enzym/Substrat-, Rezeptor/Effektor-, Lectin/Zucker-, Antikörper/Antigen-, Avidin/Biotin-, oder Streptavidin/Biotin-Wechselwirkung.

Bei einer weiteren Ausführungsform der erfindungsgemäßen Sensoroberfläche ist die Nukleinsäure eine DNA oder RNA oder ein Analogon davon.

Bei einer weiteren Ausführungsform der erfindungsgemäßen Sensoroberfläche ist die DNA oder RNA ein Oligonukleotid.

Bei einer weiteren Ausführungsform der erfindungsgemäßen Sensoroberfläche ist der Antikörper ein polyklonaler, monoklonaler, chimärer oder »Single-chain«-Antikörper oder ein funktionelles Fragment oder Derivat eines derartigen Antikörpers. Unter einem funktionellen Fragment oder Derivat eines Antikörpers wird hier jedes Fragment oder Derivat eines Antikörpers mit spezifischer Antigenbindungsfähigkeit verstanden. Diese kann sich in der Stärke von der des nativen Antikörpers unterscheiden, das Fragment oder Derivat muss dabei aber nicht notwendigerweise gleichzeitig auch immunisierende Wirkung im Körper haben.

Bei einer weiteren Ausführungsform der erfindungsgemäßen Sensoroberfläche ist das Blockierungsreagenz unter Casein, hydrolysiertem Casein, einem Tensid, Rinderserumalbumin, fötalem Kälberserum, Serum neugeborener Kälber, und Mischungen davon ausgewählt. Diese Blockierungsreagenzien sind im Handel erhältlich und beispielsweise über die Firma Sigma-Aldrich Chemie GmbH zu beziehen.

Bei einer weiteren Ausführungsform der erfindungsgemäßen Sensoroberfläche ist das Tensid unter Natriumpalmitat, Brij® 35, Brij® 58, Cetylpyridiniumchlorid-Monohydrat, Cetyltrimethylammoniumbromid, 3-(3-Cholamidopropyl)-dimethylammonio-1-propansulfonat, 3-(3-Cholamidopropyl)-dimethyl-ammonio-2-hydroxy-1-propansulfonat, Decan-1-sulfonsäure-Natriumsalz, N,N-Bis-[3-(D-gluconamido)-propyl]-deoxycholamid, Dodecan-1-sulfonsäure-Natriumsalz, Dodecyl-β-D-maltosid, 6-O-(N-Heptylcarbamoyl)-methyl-α-D-glucopyranosid, Heptan-1-sulfonsäure-Natriumsalz, N-Lauroylsarcosin-Natriumsalz, Octanoyl-N-methylglucamid, N-Nonaoyl-N-methylglucamid, Natriumcholat, Natriumdeoxycholat, Nonan-1-sulfonsäure-Natriumsalz, Nonidet P40, Octan-1-sulfonsäure-Natriumsalz, n-Octyl-β-D-glucopyranosid, Pentan-1-sulfonsäure-Natriumsalz, n-Octyl-β-D-thioglucopyranosid, Pluronic^{®} F-68, Saccharosemonolaurat, Natriumdodecylsulfat, N-Dodecyl-dimethyl-3-ammonio-1-propansulfonat, N-Tetradecyl-dimethyl-3-ammonio-1-propansulfonat, Triton^{®} X-100, und Mischungen davon ausgewählt. Diese Tenside sind im Handel erhältlich und beispielsweise über die Firma Sigma-Aldrich Chemie GmbH zu beziehen. Grundsätzlich sind alle bekannten Tenside erfindungsgemäß geeignet.

In der folgenden Tabelle werden weitere Details der obigen Tenside angegeben.

| Bezeichnung | Summenformel | Typ |
|---|---|---|
| Brij^{®} 35 | C₅₈H₁₁₈O₂₄ | nichtionisch |
| Brij^{®} 58 | C₅₆H₁₁₄O₂₁ | nichtionisch |
| Cetylpyridiniumchlorid-Monohydrat | C₂₁H₃₈ClN x H₂O | kationisch |
| Cetyltrimethylammoniumbromid | C₁₉H₄₂BrN | kationisch |
| CHAPS | C₃₂H₅₈N₂O₇S | zwitterionisch |
| CHAPSO | C₃₂H₅₈N₂O₆S | zwitterionisch |
| Decan-1-sulfonsäure-Natriumsalz | C₁₀H₂₁NaO₃S | |
| | | |
| Deoxy-BIGCHAP | C₄₂H₇₅N₃O₁₆ | nichtionisch |
| Dodecan-1-sulfonsäure-Natriumsalz | C₁₂H₃₅NaO₃S | |
| Dodecyl-β-D-maltosid | C₁₂H₃₅NaO₃S | nichtionisch |
| HECAMEG | C₁₅H₂₉NO₇ | nichtionisch |
| Heptan-1-sulfonsäure-Natriumsalz | C₇H₁₅NaO₃S x H₂O | |
| N-Lauroylsarcosin-Natriumsalz | C₁₅H₂₈NNaO₃ | anionisch |
| MEGA-8 | C₁₅H₃₁NO₆ | nichtionisch |
| MEGA-9 | C₁₆H₃₃NO₆ | nichtionisch |
| Natriumcholat | C₂₄H₃₉NaO₅ | anionisch |
| Natriumdeoxycholat | C₂₄H₃₉NaO₄ | anionisch |
| Nonan-1-sulfonsäure-Natriumsalz | C₉H₁₉NaO₃S | |
| Nonidet P40 | Mischung aus 15 Homologen | |
| Octan-1-sulfonsäure-Natriumsalz | C₈H₁₇NaO₃S | |
| n-Octyl-β-D-glucopyranosid | C₁₄H₂₈O₆ | nichtionisch |
| Pentan-1-sulfonsäure-Natriumsalz | C₅H₁₁NaO₃S | |
| n-Octyl-β-D-thioglucopyranosid | C₁₄H₂₈O₅S | |
| Pluronic^{®} F-68 | n.a. | nichtionisch |
| Saccharosemonolaurat | C₂₄H₄₄O₁₂ | nichtionisch |
| SDS (Natriumdodecylsulfat | C₂₄H₄₄O₁₂ | anionisch |
| Sulfobetain SB 12 | C₁₇H₃₇NO₃S | zwitterionisch |
| Sulfobetain SB 14 | C₁₉H₄₁NO₃S | zwitterionisch |
| Triton^{®} X-100 | | nichtionisch |
| Triton^{®} X-114 | C₃₀H₅₄O₉ | nichtionisch |
| Tween^{®} 20 | | nichtionisch |
| Tween^{®} 80 | | nichtionisch |

Abkürzungen: CHAPS (3-(3-Cholamidopropyl)-dimethylammonio-1-propansulfonat); CHAPSO (3-(3-Cholamidopropyl)-dimethyl-ammonio-2-hydroxy-1-propansulfonat); Deoxy-BIGCHAP (N,N-Bis-[3-(D-gluconamido)-propyl]-deoxycholamid); HECAMEG (6-O-(N-Heptylcarbamoyl)-methyl-α-D-glucopyranosid); MEGA-8 (Octanoyl-N-methylglucamid); MEGA-9 (N-Nonanoyl-N-methylglucamid); SDS (Natriumdodecylsulfat); Sulfobetain SB 12 (N-Dodecyl-dimethyl-3-ammonio-1-propansulfonat); Sulfobetain SB 14 (N-Tetradecyl-dimethyl-3-ammonio-1-propansulfonat).

Bei einer weiteren Ausführungsform des erfindungsgemäßen Blockierungsreagenzes ist die mindestens eine photoreaktive Gruppe unter Benzophenon oder Derivaten davon, Anthrachinon oder Derivaten davon, Thymidin oder Derivaten davon und 4-Azidobenzoesäure oder Derivaten davon ausgewählt.

Bei einer Ausführungsform des erfindungsgemäßen Verfahrens zur Herstellung des erfindungsgemäßen Blockierungsreagenzes wird die mindestens eine photoreaktive Gruppe unter Benzophenon oder Derivaten davon, Anthrachinon oder Derivaten davon, Thymidin oder Derivaten davon, und 4-Azidobenzoesäure oder Derivaten davon ausgewählt.

Die Erfindung wird im folgenden beispielhaft erläutert.

Eine geeignete Blockierungssubstanz, z.B. Casein für Polystyroloberflächen, wird mittels eines Vernetzers bzw. »Crosslinkers« mit einer photoreaktiven Gruppe versehen, z.B. 4-Azidobenzoesäure-N-hydroxy-succinimidester. Diese chemischen Gruppen erlauben es, nach der eigentlichen Blockierungsreaktion die Blockierungsreagenzien an den für sie zugänglichen Stellen kovalent zu immobilisieren. Wahlweise kann auch die Oberfläche mit photoreaktiven Gruppen versehen sein, z.B. Glas, das mit einem Benzophenonsilan beschichtet wurde. In diesem Falle kann mit dem nativen Blockierungsreagenz gearbeitet werden, und die Immobilisierung der Rezeptoren und des Blockierungsreagenzes finden durch Belichten bei geeigneten Wellenlängen statt.

Weitere erfindungsgemäß geeignete Blockierungsreagenzien sind die oben erwähnten Tenside mit einer photoreaktiven Gruppe am hydrophoben Terminus, beispielsweise Benzophenon-4-(Natriumpalmitat). Derivate auch anderer Tenside sind von einem Fachmann ohne weiteres schnell herstellbar. Diese Substanzen blockieren Bereiche, auf denen hydrophobe Wechselwirkungen stattfinden. Auch nichtionische/anionische Tenside mit photoreaktiven Gruppen sind geeignet. Ein Vorteil wäre, dass man außerdem damit stabile Lipidvesikel herstellen könnte.

### Beispiele

### Herstellung von photoreaktiven Caseinfragmenten

Vorzugsweise wird relativ niedermolekulares Casein mit einem Molekülgewicht von < 10 kD verwendet. Dieses wird in Natriumphosphatpuffer (pH-Wert 7,5) aufgelöst und mit der 20fachen molaren Menge an Crosslinker (5-Azido-2-nitrobenzoesäure-N-hydroxysuccinimidester als Stammlösung in Dimethylformamid bzw. DMF) im Dunkeln umgesetzt (Raumtemperatur, 2 Std.). Anschließend wird das Protein durch Auftrennen über eine Sephadex-Säule gereinigt und auf eine Konzentration von 1 % (Gew./Vol.) eingestellt (z.B. durch Verdünnen). Der pH-Wert der fertigen Lösung wird auf 7,0 eingestellt.

### Aufbringen auf eine Oberfläche

Das kovalente Blocken des ggf. zuvor mit Rezeptormolekülen versehenen Biosensors wird folgendermaßen durchgeführt. Die Sensoroberfläche wird 2 Std. mit dem Blockierungspuffer bei 4°C inkubiert und anschließend gründlich mit PBS-Puffer (PBS-Puffer ist phosphatgepufferte Kochsalzlösung) gewaschen. Schließlich wird mit UV-Licht (Wellenlänge ca. 300 nm) ca. 5 Minuten belichtet. Fakultativ kann die Sensoroberfläche zusätzlich noch mit einer 0,1 %igen Gelatinelösung zur besseren Stabilisierung der vorhandenen Proteine benetzt bzw. besprüht werden.

## Patentansprüche

1. Sensoroberfläche mit darauf kovalent immobilisierten spezifischen Sondenmolekülen für mindestens ein nachzuweisendes Biomolekül, wobei grundsätzlich für unspezifische Bindungen zur Verfügung stehende Stellen oder Bereiche der Sensoroberfläche durch mindestens ein kovalent daran immobilisiertes Blockierungsreagenz inaktiviert sind, erhältlich durch ein Verfahren, bei dem die Sensoroberfläche nach der kovalenten Immobilisierung der Sondenmoleküle mit dem Blockierungsreagenz beaufschlagt wird, wobei das Blockierungsreagenz mindestens einen photoreaktiven Vernetzer mit mindestens einer photoreaktiven Gruppe aufweist und die kovalente Immobilisierung desselben an der Sensoroberfläche durch geeignete Bestrahlung erfolgt.

2. Sensoroberfläche nach Anspruch 1, **dadurch gekennzeichnet, dass** die Sondenmoleküle ein adressierbares Muster bilden.

3. Sensoroberfläche nach Anspruch 1 oder 2, **dadurch gekennzeichnet, dass** die photoreaktive(n) Gruppe(n) des mindestens einen photoreaktiven Vernetzers unter Benzophenon oder Derivaten davon, Anthrachinon oder Derivaten davon, Thymidin oder Derivaten davon, und 4-Azidobenzoesäure oder Derivaten davon ausgewählt ist.

4. Sensoroberfläche nach einem der vorstehenden Ansprüche,
**dadurch gekennzeichnet, dass** die Sensoroberfläche unter Metall-, Halbmetall-, Halbmetalloxid-, Glas- und Polymeroberflächen ausgewählt ist.

5. Sensoroberfläche nach Anspruch 4, **dadurch gekennzeichnet, dass** die Metalloberfläche unter Gold- und Aluminiumoberflächen ausgewählt ist.

6. Sensoroberfläche nach Anspruch 4, **dadurch gekennzeichnet, dass** die Halbmetalloberfläche eine Siliciumoberfläche ist.

7. Sensoroberfläche nach Anspruch 4, **dadurch gekennzeichnet, dass** die Halbmetalloxidoberfläche eine Siliciumoxid- oder Aluminiumoxidoberfläche ist.

8. Sensoroberfläche nach Anspruch 4, **dadurch gekennzeichnet, dass** die Glasoberfläche eine Quarzglasoberfläche ist.

9. Sensoroberfläche nach Anspruch 4, **dadurch gekennzeichnet, dass** die Polymeroberfläche unter Oberflächen aus Cycloolefincopolymer oder Derivaten davon, Polystyrol oder Derivaten davon, Polyethylen oder Derivaten davon, Polypropylen oder Derivaten davon, Polyimid oder Derivaten davon, und Polymethylmethacrylat oder Derivaten davon ausgewählt ist.

10. Sensoroberfläche nach einem der vorstehenden Ansprüche, **dadurch gekennzeichnet, dass** das Sondenmolekül ein Partner eines spezifisch wechselwirkenden Systems von komplementären Bindungspartnern (Rezeptor/Ligand) ist.

11. Sensoroberfläche nach Anspruch 10, **dadurch gekennzeichnet, dass** das spezifisch wechselwirkende System von komplementären Bindungspartnern auf der Wechselwirkung einer Nukleinsäure mit einer komplementären Nukleinsäure, der Wechselwirkung einer Peptidnukleinsäure mit einer Nukleinsäure, der Enzym/Substrat-, Rezeptor/Effektor-, Lectin/Zucker-, Antikörper/Antigen-, Avidin/Biotin- oder Streptavidin/Biotin-Wechselwirkung beruht.

12. Sensoroberfläche nach Anspruch 11, **dadurch gekennzeichnet, dass** die Nukleinsäure eine DNA oder RNA oder ein Analogon davon ist.

13. Sensoroberfläche nach Anspruch 12, **dadurch gekennzeichnet, dass** die DNA oder RNA ein Oligonukleotid ist.

14. Sensoroberfläche nach Anspruch 11, **dadurch gekennzeichnet, dass** der Antikörper ein polyklonaler, monoklonaler, chimärer oder »Single-chain«-Antikörper oder ein funktionelles Fragment oder Derivat eines derartigen Antikörpers ist.

15. Sensoroberfläche nach einem der vorstehenden Ansprüche, **dadurch gekennzeichnet, dass** das Blockierungsreagenz unter Casein, hydrolysiertem Casein, einem Tensid, Rinderserumalbumin, fötalem Kälberserum, Serum neugeborener Kälber, und Mischungen davon ausgewählt ist.

16. Sensoroberfläche nach Anspruch 15, **dadurch gekennzeichnet, dass** das Tensid unter Natriumpalmitat, Brij^{®} 35, Brij^{®} 58, Cetylpyridiniumchlorid-Monohydrat, Cetyltrimethylammoniumbromid, 3-(3-Cholamidopropyl)-dimethylammonio-1-propansulfonat, 3-(3-Cholamidopropyl)-dimethyl-ammonio-2-hydroxy-1-propansulfonat, Decan-1-sulfonsäure-Natriumsalz, N,N-Bis-[3-(D-gluconamido)-propyl]-deoxy-cholamid, Dodecan-1-sulfonsäure-Natriumsalz, Dodecyl-β-D-maltosid, 6-O-(N-Heptylcarbamoyl)-methyl-α-D-glucopy-ranosid, Heptan-1-sulfonsäure-Natriumsalz, N-Lauroyl-sarcosin-Natriumsalz, Octanoyl-N-methylglucamid, N-Nona-oyl-N-methylglucamid, Natriumcholat, Natriumdeoxycholat, Nonan-1-sulfonsäure-Natriumsalz, Nonidet P40, Octan-1-sulfonsäure-Natriumsalz, n-Octyl-β-D-glucopyranosid, Pentan-1-sulfonsäure-Natriumsalz, n-Octyl-β-D-thioglu-copyranosid, Pluronic^{®} F-68, Saccharosemonolaurat, Natriumdodecylsulfat, N-Dodecyldimethyl-3-ammonio-1-pro-pansulfonat, N-Tetradecyldimethyl-3-ammonio-1-propansulfonat, Tritono^{®} X-100, und Mischungen davon ausgewählt ist.

17. Verfahren zum Nachweis des Vorhandenseins von Analyten in einer zu untersuchenden Probe unter Verwendung Oberflächen-gebundener Rezeptormoleküle, **dadurch gekennzeichnet, dass** eine Sensoroberfläche gemäß einem der Ansprüche 1 bis 16 verwendet wird.

18. Vorrichtung zur Verwendung in einem Verfahren gemäß Anspruch 17, **dadurch gekennzeichnet, dass** sie eine Sensoroberfläche gemäß einem der Ansprüche 1 bis 16 aufweist.

19. Kit zur Verwendung in einem Verfahren gemäß Anspruch 17, **dadurch gekennzeichnet, dass** er eine Sensoroberfläche gemäß einem der Ansprüche 1 bis 16 und gegebenenfalls Puffer und Nachweisreagenzien enthält.

20. Reagenz zur Blockierung zur Verfügung stehender Bindungsstellen mit mindestens einem photoreaktiven Vernetzer mit mindestens einer Immobilisierung an einer Sensoroberfläche.

21. Blockierungsreagenz nach Anspruch 20, **dadurch gekennzeichnet, dass** das Blockierungsreagenz unter Casein, hydrolysiertem Casein, einem Tensid, Rinderserumalbumin, fötalem Kälberserum, Serum neugeborener Kälber, und Mischungen davon ausgewählt ist.

22. Blockierungsreagenz nach Anspruch 21, **dadurch gekennzeichnet, dass** das Tensid unter Natriumpalmitat, Brij^{®} 35, Brij^{®} 58, Cetylpyridiniumchlorid-Monohydrat, Cetyltrimethylammoniumbromid, 3-(3-Cholamidopropyl)-dimethylammonio-1-propansulfonat, 3-(3-Cholamidopropyl)-dimethyl-ammonio-2-hydroxy-1-propansulfonat, Decan-1-sulfonsäure-Natriumsalz, N,N-Bis-[3-(D-gluconamido)-propyl]-deoxycholamid, Dodecan-1-sulfonsäure-Natriumsalz, Dodecyl-β-D-maltosid, 6-O-(N-Heptylcarbamoyl)-methyl-α-D-glucopyranosid, Heptan-1-sulfonsäure-Natriumsalz, N-Lau-roylsarcosin-Natriumsalz, Octanoyl-N-methylglucamid, N-Nonaoyl-N-methylglucamid, Natriumcholat, Natriumdeoxycholat, Nonan-1-sulfonsäure-Natriumsalz, Nonidet P40, Octan-1-sulfonsäure-Natriumsalz, n-Octyl-β-D-glucopyranosid, Pentan-1-sulfonsäure-Natriumsalz, n-Octyl-β-D-thioglucopyranosid, Pluronic^{®} F-68, Saccharosemonolaurat, Natriumdodecylsulfat, N-Dodecyl-dimethyl-3-ammonio-1-propansulfonat, N-Tetradecyl-dimethyl-3-ammonio-1-propansulfonat, Triton^{®} x-100, und Mischungen davon ausgewählt ist.

23. Blockierungsreagenz nach einem der Ansprüche 20 bis 22, **dadurch gekennzeichnet, dass** die mindestens eine photoreaktive Gruppe unter Benzophenon oder Derivaten davon, Anthrachinon oder Derivaten davon, Thymidin oder Derivaten davon, und 4-Azidobenzoesäure oder Derivaten davon ausgewählt ist.

24. Verfahren zur Herstellung eines Blockierungsreagenzes gemäß einem der Ansprüche 20 bis 23, **dadurch gekennzeichnet, dass** mindestens ein Blockierungsreagenz gemäß Anspruch 21 oder 22 mit mindestens einem Vernetzer umgesetzt wird, der mindestens eine photoreaktive Gruppe aufweist.

25. Verfahren nach Anspruch 24, **dadurch gekennzeichnet, dass** die mindestens eine photoreaktive Gruppe unter Benzophenon oder Derivaten davon, Anthrachinon oder Derivaten davon, Thymidin oder Derivaten davon, und 4-Azidobenzoesäure oder Derivaten davon ausgewählt wird.

26. Kit zur Herstellung einer Sensoroberfläche gemäß einem der Ansprüche 1 bis 16, **dadurch gekennzeichnet, dass** er mindestens ein Blockierungsreagenz gemäß einem der Ansprüche 20 bis 23 und gegebenenfalls eine Sensoroberfläche sowie Puffer und Reagenzien enthält.

27. Verfahren zur Herstellung einer Sensoroberfläche mit darauf kovalent immobilisierten spezifischen Sondenmolekülen für mindestens ein nachzuweisendes Biomolekül, wobei grundsätzlich für unspezifische Bindungen zur Verfügung stehende Stellen oder Bereiche der Sensoroberfläche durch mindestens ein kovalent daran immobilisiertes Blockierungsreagenz inaktiviert sind, umfassend die folgenden Schritte:
(a) Kovalente Immobilisierung der Sondenmoleküle auf der Sensoroberfläche;
(b) Kovalente Immobilisierung des mindestens einen photoreaktiven Vernetzer mit mindestens einer photoreaktiven Gruppe aufweisenden Blockierungsreagenzes auf der Sensoroberfläche durch geeignete Bestrahlung.

28. Verfahren nach Anspruch 27, **dadurch gekennzeichnet, dass** die photoreaktive(n) Gruppe(n) des mindestens einen photoreaktiven Vernetzers unter Benzophenon oder Derivaten davon, Anthrachinon oder Derivaten davon, Thymidin oder Derivaten davon, und 4-Azidobenzoesäure oder Derivaten davon ausgewählt wird.

29. Verfahren nach Anspruch 27 oder 28, **dadurch gekennzeichnet, dass** die Sensoroberfläche unter Metall-, Halbmetall-, Halbmetalloxid-, Glas- und Polymeroberflächen ausgewählt wird.

30. Verfahren nach einem der Ansprüche 27 bis 29, **dadurch gekennzeichnet, dass** das Sondenmolekül ein Partner eines spezifisch wechselwirkenden Systems von komplementären Bindungspartnern (Rezeptor/Ligand) ist.

31. Verfahren nach einem der Ansprüche 27 bis 30, **dadurch gekennzeichnet, dass** das Blockierungsreagenz unter Casein, hydrolysiertem Casein, einem Tensid, Rinderserumalbumin, fötalem Kälberserum, Serum neugeborener Kälber, und Mischungen davon ausgewählt wird.

32. Verfahren nach einem der Ansprüche 27 bis 31, **dadurch gekennzeichnet, dass** das Tensid unter Natriumpalmitat, Brij^{®} 35, Brij^{®} 58, Cetylpyridiniumchlorid-Monohydrat, Cetyltrimethylammoniumbromid, 3-(3-Cholamidopropyl)-dimethylammonio-1-propansulfonat, 3-(3-Cholamidopropyl)-dimethyl-ammonio-2-hydroxy-1-propansulfonat, Decan-1-sulfonsäure-Natriumsalz, N,N-Bis-[3-(D-gluconamido)-propyl]-deoxycholamid, Dodecan-1-sulfonsäure-Natriumsalz, Dodecyl-β-D-maltosid, 6-O-(N-Heptylcarbamoyl)-methyl-α-D-glucopy-ranosid, Heptan-1-sulfonsäure-Natriumsalz, N-Lauroylsarcosin-Natriumsalz, Octanoyl-N-methylglucamid, N-Nonaoyl-N-methylglucamid, Natriumcholat, Natriumdeoxycholat, Nonan-1-sulfonsäure-Natriumsalz, Nonidet P40, Octan-1-sulfonsäure-Natriumsalz, n-Octyl-β-D-glucopyranosid, Pentan-1-sulfonsäure-Natriumsalz, n-Octyl-β-D-thioglucopyranosid, Pluronic^{®} F-68, Saccharosemonolaurat, Natriumdodecylsulfat, N-Dodecyldimethyl-3-ammonio-1-pro-pansulfonat, N-Tetradecyldimethyl-3-ammonio-1-propansulfonat, Triton^{®} X-100, und Mischungen davon ausgewählt wird.

## Claims

1. Sensor surface with specific probe molecules for at least one biomolecule to be detected being covalently immobilized thereon, wherein sites or areas of the sensor surface being basically accessible for unspecific binding are inactivated by at least one blocking reagent covalently immobilized thereon, obtainable by a method in which the sensor surface is covered with the blocking reagent after the covalent immobilization of the probe molecules, wherein the blocking reagent has at least one photoreactive crosslinker with at least one photoreactive group and the covalent immobilization of the same on the sensor surface is effected by suitable radiation.

2. Sensor surface according to claim 1, **characterized in that** the probe molecules form an addressable pattern.

3. Sensor surface according to claim 1 or 2, **characterized in that** the photoreactive group(s) of the at least one photoreactive crosslinker is selected from benzophenone or derivatives thereof, anthrachinone or derivatives thereof, thymidine or derivatives thereof, and 4-azidobenzoic acid or derivatives thereof.

4. Sensor surface according to one of the preceding claims, **characterized in that** the sensor surface is selected from metal, semi-metal, semi-metal oxide, glass and polymer surfaces.

5. Sensor surface according to claim 4, **characterized in that** the metal surface is selected from gold and aluminium surfaces.

6. Sensor surface according to claim 4, **characterized in that** the semi-metal surface is a silicon surface.

7. Sensor surface according to claim 4, **characterized in that** the semi-metal oxide surface is a silicon oxide or aluminium oxide surface.

8. Sensor surface according to claim 4, **characterized in that** the glass surface is a silica glass surface.

9. Sensor surface according to claim 4, **characterized in that** the polymer surface is selected from surfaces of cycloolefincopolymer or derivatives thereof, polystyrene or derivatives thereof, polyethylene or derivatives thereof, polypropylene or derivatives thereof, polyimide or derivatives thereof, and polymethylmethacrylate or derivatives thereof.

10. Sensor surface according to one of the preceding claims, **characterized in that** the probe molecule is a partner of a specifically interacting system of complementary binding partners (receptor/ligand).

11. Sensor surface according to claim 10, **characterized in that** the specifically interacting system of complementary binding partners is based on the interaction of a nucleic acid with a complementary nucleic acid, the interaction of a peptide nucleic acid with a nucleic acid, the interaction between enzyme/substrate, receptor/effector, lectin/sugar, antibody/antigen, avidin/biotin or streptavidin/biotin.

12. Sensor surface according to claim 11, **characterized in that** the nucleic acid is a DNA or RNA or an analogon thereof.

13. Sensor surface according to claim 12, **characterized in that** the DNA or RNA is an oligonucleotide.

14. Sensor surface according to claim 11, **characterized in that** the antibody is a polyclonal, monoclonal, chimeric or single-chain antibody or a functional fragment or derivative of such an antibody.

15. Sensor surface according to one of the preceding claims, **characterized in that** the blocking reagent is selected from casein, hydrolyzed casein, a tenside, bovine serum albumin, fetal calf serum, newborn calf serum, and mixtures thereof.

16. Sensor surface according to claim 15, **characterized in that** the tenside is selected from sodium palmitate, Brij^{®} 35, Brij^{®} 58, cetylpyridinium chloride monohydrate, cetyltrimethylammonium bromide, 3-(3-cholamidopropyl)-dimethylammonio-1-propane sulfonate, 3-(3-cholamidopropyl)-dimethylammonio-2-hydroxy-1-propane sulfonate, decan-1-sulfonic acid-sodium salt, N,N-bis-[3-(D-gluconamido)-propyl]deoxycholamide, dodecan-1-sulfonic acid-sodium salt, dodecyl-β-D-maltoside, 6-O-(N-heptylcarbamoyl)-methyl-α-D-glucopyranoside, heptan-1-sulfonic acid-sodium salt, N-lauroyl-sarcosine-sodium salt, octanoyl-N-methylglucamide, N-nonanoyl-N-methylglucamide, sodium-cholate, sodium deoxycholate, nonan-1-sulfonic acid-sodium salt, Nonidet P40, octan-1-sulfonic acid-sodium salt, n-octyl-β-D-glycopyranoside, pentan-1-sulfonic acid-sodium salt, n-octyl-β-D-thioglucopyranoside, Pluronic^{®} F-68, saccharosemonolaurate, sodium dodecyl sulfate, N-dodecyldimethyl-3-ammonio-1-propanesulfonate, N-tetradecyldimethyl-3-ammonio-1-propanesulfonate, Triton^{®} X-100, and mixtures thereof.

17. Method for the detection of the presence of analytes in a sample to be analyzed by using surface-bound receptor molecules, **characterized in that** a sensor surface as defined in one of claims 1 to 16 is used.

18. Apparatus for use in a method as defined in claim 17, **characterized in that** it has a sensor surface as defined in one of claims 1 to 16.

19. Kit for use in a method as defined in claim 17, **characterized in that** it contains a sensor surface as defined in one of claims 1 to 16 and, optionally, buffer and detection reagents.

20. Reagent for blocking accessible binding sites having at least one photoreactive crosslinker with at least one photoreactive group for covalent immobilization on a sensor surface.

21. Blocking reagent according to claim 20, **characterized in that** the blocking reagent is selected from casein, hydrolyzed casein, a tenside, bovine serum albumin, fetal calf serum, newborn calf serum, and mixtures thereof.

22. Blocking reagent according to claim 21, **characterized in that** the tenside is selected from sodium palmitate, Brij^{®} 35, Brij^{®} 58, cetylpyridinium chloride monohydrate, cetyltrimethylammonium bromide, 3-(3-cholamidopropyl)-dimethylammonio-1-propane sulfonate, 3-(3-cholamidopropyl)-dimethylammonio-2-hydroxy-1-propane sulfonate, decan-1-sulfonic acid-sodium salt, N,N-bis-[3-(D-gluconamido)-propyl]deoxycholamide, dodecan-1-sulfonic acid-sodium salt, dodecyl-β-D-maltoside, 6-O-(N-heptylcarbamoyl)-methyl-α-D-glucopyranoside, heptan-1-sulfonic acid-sodium salt, N-lauroyl-sarcosine-sodium salt, octanoyl-N-methylglucamide, N-nonanoyl-N-methylglucamide, sodium-cholate, sodium deoxycholate, nonan-1-sulfonic acid-sodium salt, Nonidet P40, octan-1-sulfonic acid-sodium salt, n-octyl-β-D-glycopyranoside, pentan-1-sulfonic acid-sodium salt, n-octyl-β-D-thioglucopyranoside, Pluronic^{®} F-68, saccharosemonolaurate, sodium dodecyl sulfate, N-dodecyldimethyl-3-ammonio-1-propanesulfonate, N-tetradecyldimethyl-3-ammonio-1-propanesulfonate, Triton^{®} X-100, and mixtures thereof.

23. Blocking reagent according to one of claims 20 to 22, **characterized in that** the at least one photoreactive group is selected from benzophenone or derivatives thereof, anthrachinone or derivatives thereof, thymidine or derivatives thereof, and 4-azidobenzoic acid or derivatives thereof.

24. Method for the production of a blocking reagent as defined in one of claims 20 to 23, **characterized in that** at least one blocking reagent as defined in claim 21 or 22 is reacted with at least one crosslinker having at least one photoreactive group.

25. Method according to claim 24, **characterized in that** the at least one photoreactive group is selected from benzophenone or derivatives thereof, anthrachinone or derivatives thereof, thymidine or derivatives thereof, and 4-azidobenzoic acid or derivatives thereof.

26. Kit for the production of a sensor surface as defined in one of claims 1 to 16, **characterized in that** it contains at least one blocking reagent as defined in one of claims 20 to 23 and, optionally, a sensor surface as well as buffer and reagents.

27. Method for the production of a sensor surface with specific probe molecules for at least one biomolecule to be detected being covalently immobilized thereon, wherein sites or areas of the sensor surface being basically accessible for unspecific binding are inactivated by at least one blocking reagent covalently immobilized thereon, comprising the following steps:
a) covalent immobilization of the probe molecules on the sensor surface;
b) covalent immobilization of the blocking reagent having at least one photoreactive crosslinker with at least one photoreactive group on the sensor surface by suitable radiation.

28. Method according to claim 27, **characterized in that** the photoreactive group(s) of the at least one photoreactive crosslinker is selected from benzophenone or derivatives thereof, anthrachinone or derivatives thereof, thymidine or derivatives thereof, and 4-azidobenzoic acid or derivatives thereof.

29. Method according to claim 27 or 28, **characterized in that** the sensor surface is selected from metal, semi-metal, semi-metal oxide, glass and polymer surfaces.

30. Method according to one of claims 27 to 29, **characterized in that** the probe molecule is a partner of a specifically interacting system of complementary binding partners (receptor/ligand).

31. Method according to one of claims 27 to 30, **characterized in that** the blocking reagent is selected from casein, hydrolyzed casein, a tenside, bovine serum albumin, fetal calf serum, newborn calf serum, and mixtures thereof.

32. Method according to one of claims 27 to 31, **characterized in that** the tenside is selected from sodium palmitate, Brij^{®} 35, Brij^{®} 58, cetylpyridinium chloride monohydrate, cetyltrimethylammonium bromide, 3-(3-cholamidopropyl)-dimethylammonio-1-propane sulfonate, 3-(3-cholamidopropyl)-dimethylammonio-2-hydroxy-1-propane sulfonate, decan-1-sulfonic acid-sodium salt, N,N-bis-[3-(D-gluconamido)-propyl]deoxycholamide, dodecan-1-sulfonic acid-sodium salt, dodecyl-β-D-maltoside, 6-O-(N-heptylcarbamoyl)-methyl-α-D-glucopyranoside, heptan-1-sulfonic acid-sodium salt, N-lauroyl-sarcosine-sodium salt, octanoyl-N-methylglucamide, N-nonanoyl-N-methylglucamide, sodium-cholate, sodium deoxycholate, nonan-1-sulfonic acid-sodium salt, Nonidet P40, octan-1-sulfonic acid-sodium salt, n-octyl-β-D-glycopyranoside, pentan-1-sulfonic acid-sodium salt, n-octyl-β-D-thioglucopyranoside, Pluronic^{®} F-68, saccharosemonolaurate, sodium dodecyl sulfate, N-dodecyldimethyl-3-ammonio-1-propanesulfonate, N-tetradecyldimethyl-3-ammonio-1-propanesulfonate, Triton^{®} X-100, and mixtures thereof.

## Revendications

1. Une surface sensorielle avec des molécules sondes couplées de manière covalente spécifiques pour au moins une biomolécule à détecter, sur laquelle les sites ou domaines qui en principe sont disponibles pour des liaisons non spécifiques, sont inactivés par au moins un réactif de blocage qui y est immobilisé de manière covalente, obtenue par un procédé au cours duquel la surface sensorielle après l'immobilisation covalente des molécules sondes est recouverte avec du réactif de blocage, où le réactif de blocage contient au moins un liant photoréactif avec au moins un groupe photoréactif et l'immobilisation covalente de celui-ci sur la surface sensorielle est obtenue par une irradiation appropriée.

2. Une surface sensorielle selon la revendication 1 **caractérisée en ce que** les molécules sondes forment un pattern ordonné.

3. Une surface sensorielle selon la revendication 1 ou 2 **caractérisée en ce que** le (les) groupe(s) photoréactif(s) du liant photoréactif qu'il contient au moins est choisi parmi la benzophénone ou des dérivés de celle-ci, l'anthraquinone ou des dérivés de celle-ci, la thymidine ou des dérivés de celle-ci, et l'acide 4-azidobenzoïque ou de dérivé de celui-ci.

4. Une surface sensorielle selon une des revendications précédentes **caractérisée en ce que** la surface sensorielle est choisie parmi les surfaces métalliques, semi-métalliques, semi-metal oxydiques, en verre et en polymère.

5. Une surface sensorielle selon la revendication 4 **caractérisée en ce que** la surface métallique est choisie parmi les surfaces en or ou en aluminium.

6. Une surface sensorielle selon la revendication 4 **caractérisée en ce que** la surface semi-métallique est une surface de silicium.

7. Une surface sensorielle selon la revendication 4 **caractérisée en ce que** la surface semi-métal oxidique est une suface en oxyde de silicium ou en oxyde d'aluminium.

8. Une surface sensorielle selon la revendication 4 **caractérisée en ce que** la surface en verre est une surface en verre de quartz.

9. Une surface sensorielle selon la revendication 4 **caractérisée en ce que** la surface en polymère est choisie parmi les surfaces en copolymère de cyclo-oléfine ou des dérivés de celui-ci, de polystyrol ou des dérivés de celui-ci, de polyéthylène ou des dérivés de celui-ci, de poly propylène ou des dérivés de celui-ci, de polyimide ou de dérivés de celui-ci, et de polyméthacrylate ou de dérivés de celui-ci.

10. Une surface sensorielle selon une des revendications précédentes **caractérisée en ce que** la molécule sonde est un partenaire d'un système spécifique d'interaction de partenaires de liaison complémentaires (récepteur/ligand).

11. Une surface sensorielle selon la revendication 10 **caractérisée en ce que** le système spécifique d'interaction de partenaires de liaison complémentaires est basé sur l'interaction d'un acide nucléique avec l'acide nucléique complémentaire, l'interaction d'un acide nucléique peptidique et un acide nucléique, l'interaction enzyme/substrat, récepteur/effecteur, lectine/sucre, anticorps/antigène, avidine/biotine ou streptavidine/biotine.

12. Une surface sensorielle selon la revendication 11 **caractérisée en ce que** l'acide nucléique est un ADN ou ARN ou un analogue de ceux-ci.

13. Une surface sensorielle selon la revendication 12
**caractérisée en ce que** l'ADN ou l'ARN est un oligonucléotide.

14. Une surface sensorielle selon la revendication 11 **caractérisée en ce que** l'anticorps est un anticorps polyclonal, monoclonal, chimérique ou « à chaîne simple » ou un fragment ou un dérivé fonctionnel d'un tel anticorps.

15. Une surface sensorielle selon une des revendications précédentes **caractérisée en ce que** le réactif de blocage est choisi parmi la caséine, la caséine hydrolysée, un tenside, l'albumine bovine, le sérum foetal de veau, le sérum de veau nouveau-né ou un mélange de ceux-ci.

16. Une surface sensorielle selon la revendication 15 **caractérisée en ce que** le tenside est choisi parmi le palmitate de sodium, le Brij^{©} 35, le Brij^{©} 58, le monohydrate de chlorure de cétylpyridinium, le bromure de cétyltriméthylammonium, le sulfonate de 3-(3-cholamidopropyl)-diméthylammonio-1-propane, le sulfonate de 3-(3-cholamidopropyl)-diméthyl-ammonio-2-hydroxy-1-propane, le sel sodique d'acide décan-1-sulfonique, le N,N-Bis-[3-(D-gluconamido)-propyl]-déoxy-cholamide, le sel sodique d'acide dodécan-1-sulfonique, le dodécyl-β-D-maltoside, le 6-O(N-héptylcarbamoyl)-méthyl-α-D-glucopyranoside, le sel sodique d'acide héptan-1-sulfonique, le sel sodique de N-lauroyl-sarcosine, l'octanoyl-N-méthylglucamide, le N-nona-oyl-N-méthylglucamide, le cholate de sodium, le déoxycholate de sodium, le sel sodique d'acide nonan-1-sulfonique, le nonidet P40, le sel sodique d'acide octan-1-sulfonique, le n-octyl-β-D-glucopyranoside, le sel sodique d'acide pentan-1-sulfonique, le n-octyl-β-D-thioglucopyranoside, le pluronic^{©} F-68, le monolaurate de sucrose, le dodécylsulfate de sodium, le N-dodécyl-diméthyl-3-ammonio-1-propansulfonate, le N-tétradécyl-diméthyl-3-ammonio-1-propansulfonate, le Triton^{©} X-100, et les mélanges de ceux-ci.

17. Un procédé pour la détection de la présence d'analytes dans un des échantillons à analyser en utilisant une molécule réceptrice liée à la surface **caractérisé en ce que** l'on utilise une surface sensorielle selon une des revendications 1 à 16.

18. Un dispositif à utiliser dans un procédé selon la revendication 17 **caractérisé en ce que** il contient une surface sensorielle selon une des revendications 1 à 16.

19. Un kit à utiliser dans un procédé selon la revendication 17 **caractérisé en ce que** il contient une surface sensorielle selon une des revendications 1 à 16 et également un tampon et des réactifs de détection.

20. Un réactif pour bloquer les sites de liaison disponibles avec au moins un liant photoréactif avec au moins un groupe photoréactif pour une immobilisation covalente sur une surface sensorielle.

21. Un réactif de blocage selon la revendication 20 **caractérisé en ce que** le réactif de blocage est choisi parmi la caséine, la caséine hydrolysée, un tenside, l'albumine de sérum bovin, le sérum de veau foetal, le sérum de veau nouveau-né, et des mélanges de ceux-ci.

22. Un réactif de blocage selon la revendication 21 **caractérisé en ce que** le tenside est choisi parmi le palmitate de sodium, le Brij^{©} 35, le Brij^{©} 58, le monohydrate de chlorure de cétylpyridinium, le bromure de cétyltriméthylammonium, le sulfonate de 3-(3-cholamidopropyl)-diméthylammonio-1-propane, le sulfonate de 3-(3-cholamidopropyl)-diméthylammonio2-hydroxy-1-propane, le sel sodique d'acide décan-1-sulfonique, le N,N-Bis-[3-(D-gluconamido)-propyl]-déoxycholamide, le sel sodique d'acide dodécan-1-sulfonique, le dodécyl-β-D-maltoside, 6-O-(N-héptylcarbamoyl)-méthyl-α-D-glucopyranoside, le sel sodique d'acide héptan-1-sulfonique, le sel sodique de N-lauroyl-sarcosine, l'octanoyl-N-méthylglucamide, le N-nona-oyl-N-méthylglucamide, le cholate de sodium, le déoxycholate de sodium, le sel sodique d'acide nonan-1-sulfonique, le nonidet P40, le sel sodique d'acide octan-1-sulfonique, le n-octyl-β-D-glucopyranoside, le sel sodique d'acide pentan-1-sulfonique, le n-octyl-β-D-thioglucopyranoside, le pluronic^{©} F-68, le monolaurate de sucrose, le dodécylsulfate de sodium, le N-dodécyl-diméthyl-3-ammonio-1-pro-pansulfonate, le N-tétradécyl-diméthyl-3-ammonio-1-propansulfonate, le Triton^{©} X-100, et des mélanges de ceux-ci.

23. Un réactif de blocage selon une des revendications 20 à 22 **caractérisé en ce que** le groupe photoréactif qu'il contient au moins est choisi parmi la benzophénone ou des dérivés de celle-ci, l'anthraquinone ou des dérivés de celle-ci, la thymidine ou des dérivés de celles-ci, et l'acide 4-azidobenzoïque ou des dérivés de celui-ci.

24. Un procédé pour la production d'un réactif de blocage selon une des revendications 20 à 23 **caractérisé en ce que** au moins un réactif de blocage selon les revendication 21 ou 22 avec au moins un liant est utilisé, qui contient au moins un groupe photoréactif.

25. Un procédé selon la revendication 24 **caractérisé en ce que** le groupe photoréactif qu'il contient au moins est choisi parmi la benzophénone ou des dérivés de celle-ci, l'anthraquinone ou des dérivés de celle-ci, la thymidine ou des dérivés de celle-ci, et l'acide 4-azidobenzoïque ou des dérivés de celui-ci.

26. Un kit pour la production d'une surface sensorielle selon une des revendications 1 à 16 **caractérisé en ce que** il contient au moins un réactif de blocage selon une des revendications 20 à 23 et en option, une surface sensorielle et aussi un tampon et des réactifs.

27. Un procédé pour la production d'une surface sensorielle avec des molécules sondes pour au moins une biomolécule à détecter immobilisées de manière covalente à sa surface sur laquelle les sites ou domaines de la surface sensorielle qui en principe sont disponibles pour des liaisons non spécifiques, sont inactivés par au moins un réactif de blocage qui y est immobilisé de manière covalente, comprenant les étapes suivantes :
a. l'immobilisation covalente des molécules sondes sur la surface sensorielle;
b. l'immobilisation covalente du liant photoréactif qu'il contient au moins avec un réactif de blocage contenant au moins un groupe photoréactif sur la surface sensorielle par une irradiation appropriée.

28. Un procédé selon la revendication 27 **caractérisé en ce que** le (les) groupe(s) photoréactif(s) du liant photoréactif qu'il contient au moins est choisi parmi la benzophénone ou des dérivés de celle-ci, l'anthraquinone ou des dérivés de celle-ci, la thymidine ou des dérivés de celle-ci, et l'acide 4-azidobenzoïque ou des dérivés de celui-ci.

29. Un procédé selon la revendication 27 ou 28 **caractérisé en ce que** la surface sensorielle est choisi parmi les surfaces métalliques, semi-métalliques, semi-métal oxydiques, de verre et de polymères.

30. Un procédé selon une des revendications 27 à 29 **caractérisé en ce que** la molécule sonde est un partenaire d'un système d'interaction spécifique de partenaires de liaison complémentaires (récepteur/ligand).

31. Un procédé selon une des revendications 27 à 30 **caractérisé en ce que** le réactif de blocage est choisi parmi la caséine, la caséine hydrolysée, un tenside, l'albumine de sérum bovine, le sérum de veau foetal, le sérum de veau nouveau-né et des mélanges de ceux-ci.

32. Un procédé selon une des revendications 27 à 31 **caractérisé en ce que** le tenside est choisi parmi le palmitate de sodium, le Brij^{©} 35, le Brij^{©} 58, le monohydrate de chlorure de cétylpyridinium, le bromure de cétyltriméthylammonium, le sulfonate de 3-(3-cholamidopropyl)-diméthylammonio-1-propane, le sulfonate de 3-(3-cholamidopropyl)-diméthylammonio-2-hydroxy-1-propane, le sel sodique d'acide décan-1-sulfonique, le N,N-Bis-[3-(D-gluconamido)-propyl]-déoxycholamide, le sel sodique d'acide dodécan-1-sulfonique, le dodécyl-β-D-maltoside, le 6-O(N-héptylcarbamoyl)-méthyl-α-D-glucopyranoside, le sel sodique d'acide héptan-1-sulfonique, le sel sodique de N-lauroyl-sarcosine, l'octanoyl-N-méthylglucamide, le N-nona-oyl-N-méthylglucamide, le cholate de sodium, le déoxycholate de sodium, le sel sodique d'acide nonan-1-sulfonique, le nonidet P40, le sel sodique d'acide octan-1-sulfonique, le n-octyl-β-D-glucopyranoside, le sel sodique d'acide pentan-1-sulfonique, le n-octyl-β-D-thioglucopyranoside, le pluronic^{©} F-68, le monolaurate de sucrose, le dodécylsulfate de sodium, le N-dodécyl-diméthyl-3-ammonio-1-pro-pansulfonate, le N-tétradécyl-diméthyl-3-ammonio-1-propansulfonate, le Triton^{©} X-100, et les mélanges de ceux-ci.
